(19) Europäisches Patentamt
European Patent Office
Office européen des brevets

(11) EP 4 085 043 B1

(12) EUROPEAN PATENT SPECIFICATION

(45) Date of publication and mention of the grant of the patent:
**12.11.2025 Bulletin 2025/46**

(21) Application number: **20842119.8**

(22) Date of filing: **17.12.2020**

(51) International Patent Classification (IPC):
***C07C 67/343** (2006.01)* ***C07C 69/533** (2006.01)*

(52) Cooperative Patent Classification (CPC):
(C-Sets available)
**C07C 67/343;** C08F 20/00 (Cont.)

(86) International application number:
**PCT/US2020/065766**

(87) International publication number:
**WO 2021/138075 (08.07.2021 Gazette 2021/27)**

# (54) PROCESS FOR PREPARING AN ALPHA-SUBSTITUTED ACRYLATE

VERFAHREN ZUR HERSTELLUNG EINES ALPHA-SUBSTITUIERTEN ACRYLATS

PROCÉDÉ DE PRÉPARATION D'UN ACRYLATE ALPHA SUBSTITUÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2019 US 201962954941 P**

(43) Date of publication of application:
**09.11.2022 Bulletin 2022/45**

(73) Proprietor: **Dow Global Technologies LLC**
**Midland, MI 48674 (US)**

(72) Inventors:
- **AUYEUNG, Evelyn**
  **Lake Jackson, Texas 77566 (US)**
- **KRASOVSKIY, Arkady L.**
  **Lake Jackson, Texas 77566 (US)**
- **MURPHY, Jaclyn**
  **Ashland, Massachusetts 01721 (US)**
- **STUBBERT, Bryan D.**
  **Midland, Michigan 48674 (US)**
- **DAVIS, Anna V.**
  **Auburn, Michigan 48611 (US)**
- **CUMMINS, Clark H.**
  **Midland, Michigan 48674 (US)**

(74) Representative: **Boult Wade Tennant LLP**
**Salisbury Square House**
**8 Salisbury Square**
**London EC4Y 8AP (GB)**

(56) References cited:
- **VETTEL S ET AL: "A New Preparation of Diorganozincs from Olefins via a Nickel Catalyzed Hydrozincation", TETRAHEDRON LETTERS, ELSEVIER, AMSTERDAM , NL, vol. 36, no. 7, 13 February 1995 (1995-02-13), pages 1023 - 1026, XP004028638, ISSN: 0040-4039, DOI: 10.1016/0040-4039(94)02438-H**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 67/343, C07C 69/533**

## Description

BACKGROUND

An article taken from Tetrahedron Letters, Volume 36, Issue 7, 13 February 1995, pages 1023 to 1026 relates to a preparation of diorganozincs from olefins via a nickel catalyzed hydrozincation.

Alpha-substituted acrylates, such as alpha-(alkyl) acrylates or alpha-(polymeryl) acrylates, are important intermediates for preparing useful polymeric materials. Synthetic routes for the preparation of alpha-substituted acrylates and bisacrylates from organozinc reagents are known in the art. However, such routes employ transmetallation of organozinc compounds to organocopper compounds, and the direct reaction of organozinc compounds with halomethylacrylates is not known. The present disclosure addresses such a need by demonstrating a direct reaction of organozinc compounds with alpha-(halomethyl) acrylates to synthesize alpha-(alkyl) and alpha-(polymeryl) acrylates.

SUMMARY

The present disclosure is directed to a process for preparing an alpha-substituted acrylate, the process comprising:

a) combining starting materials comprising an alpha-(halomethyl) acrylate and an organozinc compound of the formula $R_2Zn$,
thereby forming a product comprising the alpha-substituted acrylate, wherein:

the alpha-substituted acrylate has the formula (I):

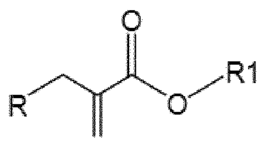
(I);

the alpha-(halomethyl) acrylate has the formula (II):

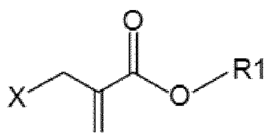
(II);

each R independently is a C1-C26 hydrocarbyl group or a polyolefinyl group;
each R1 independently is a C1-C30 hydrocarbyl group; and
X is a halogen, wherein the process excludes any transmetallation step or reaction.

BRIEF DESCRIPTION OF DRAWINGS

FIGS. 1A and 1B provide the $^{1}H$ NMR and $^{13}C$ NMR spectra, respectively, for Example 1.
FIGS. 1C and 1D provide the GCMS spectra for Example 1.
FIG. 2 provides the NMR spectra for Example 2.
FIGS. 3A and 3B provide the NMR spectra for Example 3.
FIGS. 4A and 4B provide the NMR spectra for Example 4.

DETAILED DESCRIPTION

*Definitions*

All references to the Periodic Table of the Elements herein shall refer to the Periodic Table of the Elements, published and copyrighted by CRC Press, Inc., 2003. Also, any references to a Group or Groups shall be to the Group or

Groups reflected in this Periodic Table of the Elements using the IUPAC system for numbering groups.

Unless stated to the contrary, implicit from the context, or customary in the art, all parts and percents are based on weight.

The numerical ranges disclosed herein include all values from, and including, the lower and upper value. For ranges containing explicit values (e.g., 1, or 2, or 3 to 5, or 6, or 7), any subrange between any two explicit values is included (e.g., 1 to 2; 2 to 6; 5 to 7; 3 to 7; 5 to 6; etc.). The numerical ranges disclosed herein further include the fractions between any two explicit values.

The terms "comprising," "including," "having" and their derivatives are not intended to exclude the presence of any additional component, step or procedure, whether or not the same is specifically disclosed. In contrast, the term "consisting essentially of" excludes from the scope of any succeeding recitation any other component, step, or procedure, excepting those that are not essential to operability. The term "consisting of" excludes any component, step, or procedure not specifically delineated or listed. The term "or," unless stated otherwise, refers to the listed members individually as well as in any combination.

As used herein, the terms "hydrocarbyl," "hydrocarbyl group," and like terms refer to compounds composed entirely of hydrogen and carbon, including aliphatic, aromatic, acyclic, cyclic, polycyclic, branched, unbranched, saturated, and unsaturated compounds. The terms "hydrocarbyl," "hydrocarbyl group," "alkyl," "alkyl group," "aryl," "aryl group," and like terms are intended to include every possible isomer, including every structural isomer or stereoisomer.

The term "cyclic" refers to a series of atoms in a polymer or compound where such a series includes one or more rings. Accordingly, the term "cyclic hydrocarbyl group" refers to a hydrocarbyl group that contains one or more rings. A "cyclic hydrocarbyl group," as used herein, may contain acyclic (linear or branched) portions in addition to the one or more rings.

"Transmetalation" or "transmetallation" refers to an organometallic reaction involving the transfer of ligands from one metal to another.

The term "catalyst" is used interchangeably with "procatalyst," "precatalyst," "catalyst precursor," "transition metal catalyst," "transition metal catalyst precursor." "polymerization catalyst," "polymerization catalyst precursor," "transition metal complex," "transition metal compound," "metal complex," "metal compound," "complex." "metal-ligand complex," and like terms.

"Co-catalyst" refers to a compound that can activate certain procatalysts to form an active catalyst capable of polymerization of unsaturated monomers. The term "co-catalyst" is used interchangeably with "activator" and like terms.

"Active catalyst," "active catalyst composition." and like terms refer to a transition metal compound that is, with or without a co-catalyst, capable of polymerization of unsaturated monomers. An active catalyst may be a "procatalyst" that becomes active to polymerize unsaturated monomers without a co-catalyst. Alternatively, an active catalyst may a "procatalyst" that becomes active, in combination with a co-catalyst, to polymerize unsaturated monomers.

The term "polymer" refers to a material prepared by reacting *(i.e.,* polymerizing) a set of monomers. wherein the set is a homogenous *(i.e.,* only one type) set of monomers or a heterogeneous (*i.e.*, more than one type) set of monomers. The term polymer as used herein includes the term "homopolymer," which refers to polymers prepared from a homogenous set of monomers, and the term "interpolymer" as defined below.

The term "interpolymer" refers to a polymer prepared by the polymerization of at least two different types of monomers. This term include both "copolymers," *i.e.,* polymers prepared from two different types of monomers, and polymers prepared from more than two different types of monomers, e.g., terpolymers, tetrapolymers, etc. This term also embraces all forms of interpolymers, such as random, block, homogeneous, heterogeneous, etc.

A "polyolefin" is a polymer produced from the polymerization of an olefin as a monomer, where an olefin monomer is a linear, branched, or cyclic compound of carbon and hydrogen having at least one double bond. Accordingly, the term "polyolefin," as used herein, includes and covers the terms "ethylene-based polymer," "propylene-based polymer," "ethylene homopolymer." "propylene homopolymer," "ethylene/alpha-olefin interpolymer," "ethylene/alpha-olefin copolymer," "ethylene/alpha-olefin multiblock interpolymer," "block composite." "specified block composite," "crystalline block composite." "propylene/alpha-olefin interpolymer," and "propylene/alpha-olefin copolymer."

An "ethylene-based polymer" is a polymer that contains a majority amount of polymerized ethylene, based on the weight of the polymer, and, optionally, may further contain polymerized units of at least one comonomer. An "ethylene-based interpolymer" is an interpolymer that contains, in polymerized form, a majority amount of ethylene, based on the weight of the interpolymer, and further contains polymerized units of at least one comonomer. An "ethylene homopolymer" is a polymer that comprises repeating units derived from ethylene but does not exclude residual amounts of other components.

The term "ethylene/alpha-olefin interpolymer," as used herein, refers to a polymer that comprises, in polymerized form. a majority weight percent of ethylene (based on the weight of the interpolymer), and at least one comonomer that is an alpha-olefin. The ethylene/alpha-olefin interpolymer may be a random or block interpolymer. The terms "ethylene/alpha-olefin copolymer" and "ethylene/alpha-olefin multi-block interpolymer" are covered by the term "ethylene/alpha-olefin interpolymer."

The term "ethylene/alpha-olefin copolymer," as used herein, refers to a copolymer that comprises, in polymerized form, a majority weight percent of ethylene (based on the weight of the copolymer). and a comonomer that is an alpha-olefin, where ethylene and the alpha-olefin are the only two monomer types. The ethylene/alpha-olefin copolymer may be a random or block copolymer.

The term "ethylene/alpha-olefin multi-block interpolymer" or "olefin block copolymer," as used herein, refers to an interpolymer that includes ethylene and one or more copolymerizable alpha-olefin comonomers in polymerized form, characterized by multiple blocks or segments of two or more (preferably three or more) polymerized monomer units, the blocks or segments differing in chemical or physical properties. Specifically. this term refers to a polymer comprising two or more (preferably three or more) chemically distinct regions or segments (referred to as "blocks") joined in a linear manner, that is, a polymer comprising chemically differentiated units which are joined (covalently bonded) end-to-end with respect to polymerized functionality, rather than in pendent or grafted fashion. The blocks differ in the amount or type of comonomer incorporated therein, the density, the amount of crystallinity, the type of crystallinity (e.g., polyethylene versus polypropylene), the crystallite size attributable to a polymer of such composition, the type or degree of tacticity (isotactic or syndiotactic), region-regularity or region-irregularity, the amount of branching, including long chain branching or hyper-branching, the homogeneity, and/or any other chemical or physical property. The block copolymers are characterized by unique distributions of both polymer polydispersity (PDI or Mw/Mn) and block length distribution, e.g., based on the effect of the use of a shuttling agent(s) in combination with catalyst systems. Non-limiting examples of the olefin block copolymers of the present disclosure, as well as the processes for preparing the same, are disclosed in U.S. Patent Nos. 7,858,706 B2, 8,198,374 B2, 8,318,864 B2, 8,609,779 B2, 8,710,143 B2, 8,785,551 B2, and 9,243,090 B2.

The term "block composite" ("BC") refers to a polymer comprising three polymer components: (i) an ethylene-based polymer (EP) having an ethylene content from 10 mol% to 90 mol% (a soft copolymer), based on the total moles of polymerized monomer units in the ethylene-based polymer (EP); (ii) an alpha-olefin-based polymer (AOP) having an alpha-olefin content of greater than 90 mol% (a hard copolymer), based on the total moles of polymerized monomer units in the alpha-olefin-based polymer (AOP); and (iii) a block copolymer (diblock copolymer) having an ethylene block (EB) and an alpha-olefin block (AOB); wherein the ethylene block of the block copolymer is the same composition as the EP of component (i) of the block composite and the alpha-olefin block of the block copolymer is the same composition as the AOP of component (ii) of the block composite. Additionally, in the block composite, the compositional split between the amount of EP and AOP will be essentially the same as that between the corresponding blocks in the block copolymer. Non-limiting examples of the block composites of the present disclosure, as well as processes for preparing the same, are disclosed in U.S. Patent Nos. 8,686,087 and 8,716,400.

The term "specified block composite" ("SBC") refers to a polymer comprising three polymer components: (i) an ethylene-based polymer (EP) having an ethylene content from 78 mol% to 90 mol% (a soft copolymer), based on the total moles of polymerized monomer units in the ethylene-based polymer (EP); (ii) an alpha-olefin-based polymer (AOP) having an alpha-olefin content of from 61 mol% to 90 mol% (a hard copolymer), based on the total moles of polymerized monomer units in the alpha-olefin-based polymer (AOP); and (iii) a block copolymer (diblock copolymer) having an ethylene block (EB) and an alpha-olefin block (AOB); wherein the ethylene block of the block copolymer is the same composition as the EP of component (i) of the specified block composite and the alpha-olefin block of the block copolymer is the same composition as the AOP of component (ii) of the specified block composite. Additionally, in the specified block composite, the compositional split between the amount of EP and AOP will be essentially the same as that between the corresponding blocks in the block copolymer. Non-limiting examples of the specified block composites of the present disclosure, as well as processes for preparing the same, are disclosed in WO 2017/044547.

The term "crystalline block composite" ("CBC") refers to polymers comprising three components: (i) a crystalline ethylene based polymer (CEP) having an ethylene content of greater than 90 mol%, based on the total moles of polymerized monomer units in the crystalline ethylene based polymer (CEP); (ii) a crystalline alpha-olefin based polymer (CAOP) having an alpha-olefin content of greater than 90 mol%, based on the total moles of polymerized monomer units in the crystalline alpha-olefin based copolymer (CAOP); and (iii) a block copolymer comprising a crystalline ethylene block (CEB) and a crystalline alpha-olefin block (CAOB); wherein the CEB of the block copolymer is the same composition as the CEP of component (i) of the crystalline block composite and the CAOB of the block copolymer is the same composition as the CAOP of component (ii) of the crystalline block composite. Additionally, in the crystalline block composite, the compositional split between the amount of CEP and CAOP will be essentially the same as that between the corresponding blocks in the block copolymer. Non-limiting examples of the crystalline block composites of the present disclosure, as well as the processes for preparing the same, are disclosed in US Pat. No. 8,822,598 B2 and WO 2016/01028961 A1.

A "propylene-based polymer" is a polymer that contains a majority amount of polymerized propylene, based on the weight of the polymer, and, optionally, may further contain polymerized units of at least one comonomer. A "propylene-based interpolymer" is an interpolymer that contains, in polymerized form, a majority amount of propylene, based on the weight of the interpolymer, and further contains polymerized units of at least one comonomer. A "propylene homopolymer" is a polymer that comprises repeating units derived from propylene but does not exclude residual amounts of other

components.

The term "propylene/alpha-olefin interpolymer," as used herein, refers to a polymer that comprises, in polymerized form, a majority weight percent of propylene (based on the weight of the interpolymer), and at least one comonomer that is an alpha-olefin (where ethylene is considered an alpha-olefin). The propylene/alpha-olefin interpolymer may be a random or block interpolymer. The term "propylene/alpha-olefin interpolymer" includes the term "propylene/alpha-olefin copolymer."

The term "propylene/alpha-olefin copolymer," as used herein, refers to a copolymer that comprises. in polymerized form, a majority weight percent of propylene (based on the weight of the copolymer), and a comonomer that is an alpha-olefin, wherein propylene and the alpha-olefin are the only two monomer types. The propylene/alpha-olefin copolymer may be a random or block copolymer.

The terms "polymeryl," "polymeryl group" and like terms refer to a polymer missing one hydrogen.

The terms "polyolefinyl," "polyolefinyl group" and like terms refer to a polyolefin missing one hydrogen.

*Alpha-(halomethyl) acrylate*

The starting materials of step a) of the process of the present disclosure comprise an alpha-(halomethyl) acrylate. In certain embodiments, the alpha-(halomethyl) acrylate has the formula (II):

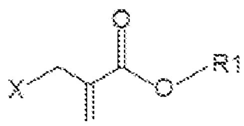

(II),

wherein:

X is a halogen; and
R1 is a C1-C30 hydrocarbyl group.

In certain embodiments, X is a halogen and is selected from the group consisting of fluoride. chloride, bromide and iodide.

In certain embodiments, R1 is a C1-C30 hydrocarbyl group that may be linear branched, or cyclic. In further embodiments, R1 is a C1-C30 alkyl group that may be linear, branched, or cyclic. For example, R1 may be a linear, branched, or cyclic alkyl group comprising from 1 to 30 carbon atoms, or from 1 to 20 carbon atoms, or from 1 to 10 carbon atoms, or from 1 to 8 carbon atoms, or from 1 to 3 carbon atoms.

*Organozinc Compound*

The starting materials of step a) of the process of the present disclosure comprise an organozinc compound of the formula $R_2Zn$, wherein each R independently is a C1-C26 hydrocarbyl group or a polyolefinyl group.

In certain embodiments, each R of the organozinc compound of the formula $R_2Zn$ independently is a C1-C26 hydrocarbyl group. In certain embodiments, each R of the organozinc compound of the formula $R_2Zn$ independently is a C1-C26 hydrocarbyl group that may be linear, branched, or cyclic. In further embodiments, each R of the organozinc compound of the formula $R_2Zn$ independently is a C1-C26 alkyl group that may be linear, branched, or cyclic. For example, each R of the organozinc compound of the formula $R_2Zn$ may independently be a linear, branched, or cyclic alkyl group comprising from 1 to 26 carbon atoms, or from 1 to 10 carbon atoms, or from 1 to 8 carbon atoms.

In some embodiments, each R of the organozinc compound of the formula $R_2Zn$ independently is a polyolefinyl group. In further embodiments, each R of the organozinc compound of the formula $R_2Zn$ independently is a polyolefinyl group, which can be defined by the properties of R-H, wherein R-H has a number average molecular weight of greater than 365 g/mol. In further embodiments, each R of the organozinc compound of the formula $R_2Zn$ independently is a polyolefinyl group, which can be defined by the properties of R-H, wherein R-H has a number average molecular weight from greater than 365 g/mol to 10,000,000 g/mol, or from greater than 365 g/mol to 5,000,000 g/mol, or from greater than 365 g/mol to 1,000,000 g/mol, or from greater than 365 g/mol to 750,000 g/mol. or from greater than 365 g/mol to 500,000 g/mol. or from greater than 365 g/mol to 250,000 g/mol.

In further embodiments, each R of the organozinc compound of the formula $R_2Zn$ independently is a polyolefinyl group. which can be defined by the properties of R-H, wherein R-H has a density from 0.850 to 0.965 g/cc, or from 0.860 to 0.950 g/cc, or from 0.865 to 0.925 g/cc.

**In** further embodiments, each R of the organozinc compound of the formula $R_2Zn$ independently is a polyolefinyl group, which can be defined by the properties of R-H. wherein R-H has a melt index (12) from 0.01 to 2,000 g/10 minutes, or

from 0.01 to 1,500 g/10 minutes. or from 0.1 to 1,000 g/10 minutes. or from 0.1 to 500 g/10 minutes, or from 0.1 to 100 g/10 minutes.

In further embodiments, each R of the organozinc compound of the formula $R_2Zn$ independently is a polyolefinyl group, which can be defined by the properties of R-H. wherein R-H has a number average molecular weight distribution (Mw/Mn or PDI) from 1 to 10, or from 1 to 7, or from 1 to 5, or from 2 to 4.

In certain embodiments, each R of the organozinc compound of the formula $R_2Zn$ independently is an ethylene homopolymeryl group comprising units derived from ethylene.

In certain embodiments, each R of the organozinc compound of the formula $R_2Zn$ independently is an ethylene/alpha-olefin interpolymeryl group comprising units derived from ethylene and at least one C3-C30 alpha-olefin. The C3-C30 alpha-olefin may be, for example, 1-butene, 4-methyl-1-pentene, 1-hexene, 1-octene, 1-decene, 1-dodecene, 1-tetradecene. 1-hexadecene, or 1-octadecene.

In certain embodiments, each R of the organozinc compound of the formula $R_2Zn$ independently is an ethylene/alpha-olefin copolymeryl group comprising units derived from ethylene and a C3-C30 alpha-olefin. The C3-C30 alpha-olefin may be, for example, propylene, 1-butene, 4-methyl-1-pentene, 1-hexene, 1-octene, 1-decene, 1-dodecene, 1-tetradecene, 1-hexadecene, or 1-octadecene.

In certain embodiments, each R of the organozinc compound of the formula $R_2Zn$ independently is an ethylene/alpha-olefin multi-block interpolymeryl group or olefin block copolymeryl group as defined herein.

In further embodiments, each R of the organozinc compound of the formula $R_2Zn$ independently is a polymeryl group of a block composite. a specified block composite, or a crystalline block composite, as defined herein.

In certain embodiments, each R of the organozinc compound of the formula $R_2Zn$ independently is a propylene homopolymeryl group comprising units derived from propylene.

In certain embodiments, each R of the organozinc compound of the formula $R_2Zn$ independently is a propylene/alpha-olefin interpolymeryl group comprising units derived from propylene and at least one comonomer that is ethylene or a C3-C30 alpha-olefin. The C3-C30 alpha-olefin may be, for example, propylene, 1-butene, 4-methyl-1-pentene, 1-hexene, 1-octene, 1-decene, 1-dodecene, 1-tetradecene, 1-hexadecene, or 1-octadecene.

In certain embodiments, each R of the organozinc compound of the formula $R_2Zn$ independently is a propylene/alpha-olefin copolymeryl group comprising units derived from propylene and a comonomer that is ethylene or a C3-C30 alpha-olefin. The C3-C30 alpha-olefin may be, for example, propylene, 1-butene, 4-methyl-1-pentene, 1-hexene, 1-octene, 1-decene, 1-dodecene, 1-tetradecene, 1-hexadecene, or 1-octadecene.

Regarding the embodiments wherein each R of the organozinc compound of the formula $R_2Zn$ independently is a polyolefinyl group, the organozinc compound of the formula $R_2Zn$ may be prepared by a process (a1), wherein the process (a1) comprises combining starting materials comprising:

i) an olefin monomer component;
ii) a catalyst; and
iii) a chain shuttling agent of the formula $J_2Zn$, wherein each J independently is a C1-C20 hydrocarbyl group,

thereby forming a solution or slurry comprising the organozinc compound of the formula $R_2Zn$.

Starting material i), the olefin monomer component, comprises one or more olefin monomers. Suitable olefin monomers include straight chain or branched alpha-olefins of 2 to 30 carbon atoms, alternatively 2 to 20 carbon atoms, such as ethylene, propylene, 1-butene, 3-methyl-1-butene, 1-pentene, 1-hexene, 4-methyl-1-pentene, 3-methyl-1-pentene, 1-octene, 1-decene, 1-dodecene, 1-tetradecene, 1-hexadecene, 1-octadecene, and 1-eicosene; cycloolefins of 3 to 30, alternatively 3 to 20 carbon atoms such as cyclopentene, cycloheptene, norbornene, 5-methyl-2-norbornene, tetracyclododecene, and 2-methyl-1,4,5,8-dimethano-1,2,3,4,4a,5,8,8a-octahydronaphthalene. Suitable olefin monomers are disclosed for example, at col. 16, lines 5-36 of U.S. Patent 7,858,706 and at col. 12, lines 7 to 41 of U.S. Patent 8,053,529. In certain embodiments, starting material i) may comprise ethylene and optionally one or more olefin monomers other than ethylene, such as propylene or 1-octene.

Regarding starting material ii), suitable catalysts include any compound or combination of compounds that is adapted for preparing polymers of the desired composition or type. One or more catalysts may be used. For example, first and second olefin polymerization catalysts may be used for preparing polymers differing in chemical or physical properties. Both heterogeneous and homogeneous catalysts may be employed. Examples of heterogeneous catalysts include Ziegler-Natta compositions, especially Group 4 metal halides supported on Group 2 metal halides or mixed halides and alkoxides and chromium or vanadium based catalysts. Alternatively, for ease of use and for production of narrow molecular weight polymer segments in solution, the catalysts may be homogeneous catalysts comprising an organometallic compound or metal complex, such as compounds or complexes based on metals selected from Groups 3 to 15 or the Lanthanide series of the Periodic Table of the Elements. Starting material ii) may further comprise a cocatalyst in addition to the catalyst. The cocatalyst may be a cation forming co-catalyst, a strong Lewis Acid, or combination thereof. Suitable catalysts and cocatalysts are disclosed, for example, at col. 19, line 45 to col. 51, line 29 of U.S. Patent 7,858,706, and col.

16, line 37 to col. 48, line 17 of U.S. Patent 8,053,529. Suitable procatalysts that may also be added include but are not limited to those disclosed in PCT Publications WO 2005/090426, WO 2005/090427, WO 2007/035485, WO 2009/012215, WO 2014/105411, WO 2017/173080, U.S. Patent Publication Nos. 2006/0199930, 2007/0167578, 2008/0311812, and U.S. Patent Nos. 7,355,089 B2, 8,058,373 B2, and 8,785,554 B2.

Regarding starting material iii), the chain shuttling agent has the formula $J_2Zn$, where each J is independently a hydrocarbyl group of 1 to 20 carbon atoms. The hydrocarbyl group for J has 1 to 20 carbon atoms, alternatively 2 to 12 carbon atoms. The hydrocarbyl group for J may be an alkyl group, which may be linear or branched. J may be an alkyl group exemplified by ethyl, propyl, octyl, and combinations thereof. Suitable chain shuttling agents include dialkyl zinc compounds, such as diethylzinc. Suitable chain shuttling agents are disclosed at col. 16, line 37 to col. 19, line 44 of U.S. Patent 7,858,706 and col. 12, line 49 to col. 14, line 40 of U.S. Patent 8,053,529.

The starting materials for preparing the organozinc compound of the formula $R_2Zn$ may optionally further comprise one or more additional starting materials selected from: iv) a solvent, vi) a scavenger, vii) an adjuvant, and viii) a polymerization aid. Toluene and Isopar™ E are examples of solvents for starting material iv). Isopar™ E is an isoparaffin fluid, typically containing less than 1 ppm benzene and less than 1 ppm sulfur, which is commercially available from ExxonMobil Chemical Company.

The process conditions and equipment for preparing the organozinc compound of the formula $R_2Zn$ are known in the art and are disclosed, for example in U.S. Patent 7,858,706 and U.S. Patent 8,053,529. For example, the process (a1) may be characterized as polymerization that is desirably carried out as a continuous polymerization, preferably a continuous, solution polymerization, in which catalyst components, shuttling agent(s), monomers, and optionally solvent, adjuvants, scavengers, and polymerization aids are continuously supplied to the reaction zone and polymer product continuously removed there from. Within the scope of the terms "continuous" and "continuously" as used in this context are those processes in which there are intermittent additions of reactants and removal of products at small regular or irregular intervals, so that, over time, the overall process is substantially continuous.

The polymerization can be advantageously employed as a high pressure, solution, slurry, or gas phase polymerization process. For a solution polymerization process it is desirable to employ homogeneous dispersions of the catalyst components in a liquid diluent in which the polymer is soluble under the polymerization conditions employed. One such process utilizing an extremely fine silica or similar dispersing agent to produce such a homogeneous catalyst dispersion where either the metal complex or the cocatalyst is only poorly soluble is disclosed in U.S. Pat. No. 5,783,512. A solution process to prepare the novel polymers of the present invention, especially a continuous solution process is preferably carried out at a temperature between 80° C. and 250° C., more preferably between 100° C. and 210° C., and most preferably between 110° C. and 210° C. A high pressure process is usually carried out at temperatures from 100° C. to 400° C. and at pressures above 50 MPa (500 bar) . A slurry process typically uses an inert hydrocarbon diluent and temperatures of from 0° C. up to a temperature just below the temperature at which the resulting polymer becomes substantially soluble in the inert polymerization medium. Preferred temperatures in a slurry polymerization are from 30° C., preferably from 60° C. up to 115° C., preferably up to 100° C. Pressures typically range from 100 kPa (atmospheric) to 3.4 MPa (500 psi) .

*Preparing an alpha-substituted acrylate*

The present process is directed to preparing an alpha-substituted acrylate. In certain embodiments, the alpha-substituted acrylate has the formula (I):

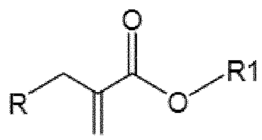

(I);

wherein R is a C1-C26 hydrocarbyl group or a polyolefinyl group; and
R1 is a C1-C30 hydrocarbyl group.

Each of the R and R1 groups of the alpha-substituted acrylate of the formula (1) is the same as and includes all embodiments of the R group of the organozinc compound of the formula $R_2Zn$ and the R1 group of the alpha-(halomethyl) acrylate of the formula (II), respectively. Indeed, the process of the present disclosure relates to a nucleophilic substitution reaction whereby X, the halogen, is a leaving group that is replaced by an R of the organozinc compound of the formula $R_2Zn$.

**In** certain embodiments, step a) of the process of the present disclosure may be performed neat. In further embodiments, the starting materials in step a) of the process of the present disclosure further comprise a hydrocarbon

solvent. In further embodiments, the starting materials in step a) of the process of the present disclosure further comprise a hydrocarbon solvent that is a non-aromatic hydrocarbon solvent.

In some embodiments, step a) of the process of the present disclosure is performed at a temperature that is above the melting temperature of the R group as defined herein. For example and without limitation. step a) of the process of the present disclosure may be performed at a temperature from 15°C to 100°C.

In some embodiments, the ratio of the alpha-(halomethyl) acrylate to the organozinc compound of the formula $R_2Zn$ in step a) is 15:1, or 10:1, or 8:1, or 6:1. or 4:1, or 2:1, or 1:1.

In preferred embodiments, the process of the present disclosure excludes any transmetallation step or reaction.

In certain embodiments, step a) of the process of the present disclosure is uncatalyzed. In further embodiments, step a) of the process of the present disclosure may be catalyzed by one or more organocatalysts. An organocatalysts is a catalyst that excludes any metal elements. Accordingly. in certain embodiments, the starting materials in step a) of the present process further comprises an organocatalyst. For example, the starting materials in step a) of the present process further comprises a nitrogen containing heterocycle as disclosed in WO 2019/182992. The nitrogen containing heterocycle can be, for example and without limitation, N-methyl imidazole.

TEST METHODS

*Density:*

Density is measured in accordance with ASTM D-792, Method B.

*Melt Index:*

Melt index ($I_2$) is measured in accordance with ASTM D-1238, which is incorporated herein by reference in its entirety, Condition 190 °C/2.16 kg, and was reported in grams eluted per 10 minutes.

*GPC:*

Sample polymers are tested for their properties via GPC according to the following.

A high temperature Gel Permeation Chromatography system (GPC IR) consisting of an Infra-red concentration detector (IR-5) from PolymerChar Inc (Valencia, Spain) was used for Molecular Weight (MW) and Molecular Weight Distribution (MWD) determination. The carrier solvent was 1,2,4-trichlorobenzene (TCB). The auto-sampler compartment was operated at 160 °C, and the column compartment was operated at 150 °C. The columns used were four Polymer Laboratories Mixed A LS, 20 μm (micron) columns. The chromatographic solvent (TCB) and the sample preparation solvent were from the same solvent source with 250 ppm of butylated hydroxytoluene (BHT) and nitrogen sparged. The samples were prepared at a concentration of 2 mg/mL in TCB. Polymer samples were gently shaken at 160 °C for 2 hours. The injection volume was 200 μl, and the flow rate was 1.0 ml/minute.

Calibration of the GPC column set was performed with 21 narrow molecular weight distribution polystyrene standards. The molecular weights of the standards ranged from 580 to 8,400,000 g/mol, and were arranged in 6 "cocktail" mixtures, with at least a decade of separation between individual molecular weights.

The GPC column set was calibrated before running the examples by running twenty-one narrow molecular weight distribution polystyrene standards. The molecular weight (Mw) of the standards ranges from 580 to 8,400,000 grams per mole (g/mol), and the standards were contained in 6 "cocktail" mixtures. Each standard mixture had at least a decade of separation between individual molecular weights. The standard mixtures were purchased from Polymer Laboratories (Shropshire, UK). The polystyrene standards were prepared at 0.025 g in 50 mL of solvent for molecular weights equal to or greater than 1,000,000 g/mol and 0.05 g in 50 mL of solvent for molecular weights less than 1,000,000 g/mol. The polystyrene standards were dissolved at 80 °C with gentle agitation for 30 minutes. The narrow standards mixtures were run first and in order of decreasing highest molecular weight (Mw) component to minimize degradation. The polystyrene standard peak molecular weights were converted to polyethylene Mw using the Mark-Houwink constants. Upon obtaining the constants, the two values were used to construct two linear reference conventional calibrations for polyethylene molecular weight and polyethylene intrinsic viscosity as a function of elution column.

The polystyrene standard peak molecular weights were converted to polyethylene molecular weights using the following equation (as described in Williams and Ward, J. Polym. Sci., Polym. Let., 6, 621 (1968)):

$$M_{polyethylene} = A(M_{polystyrene})^B \qquad (1)$$

Here B has a value of 1.0. and the experimentally determined value of A is around 0.41.

A third order polynomial was used to fit the respective polyethylene-equivalent calibration points obtained from equation (1) to their observed elution volumes of polystyrene standards.

Number, weight, and z-average molecular weights were calculated according to the following equations:

$$\overline{Mn} = \frac{\sum^i Wf_i}{\sum^i \left( {Wf_i}/{M_i} \right)} \qquad (2)$$

$$\overline{Mw} = \frac{\sum^i (Wf_i * M_i)}{\sum^i Wf_i} \qquad (3)$$

$$\overline{Mz} = \frac{\sum^i (Wf_i * M_i^2)}{\sum^i (Wf_i * M_i)} \qquad (4)$$

Where. $Wf_i$ is the weight fraction of the i-th component and $M_i$ is the molecular weight of the i-th component.

The MWD was expressed as the ratio of the weight average molecular weight (Mw) to the number average molecular weight (Mn).

The accurate A value was determined by adjusting A value in equation (1) until Mw calculated using equation (3) and the corresponding retention volume polynomial, agreed with the known Mw value of 120,000 g/mol of a standard linear polyethylene homopolymer reference.

The GPC system consists of a Waters (Milford, Mass.) 150 °C high temperature chromatograph (other suitable high temperatures GPC instruments include Polymer Laboratories (Shropshire, UK) Model 210 and Model 220) equipped with an on-board differential refractometer (RI). Additional detectors could include an IR4 infra-red detector from Polymer ChAR (Valencia, Spain), Precision Detectors (Amherst, Mass.) 2-angle laser light scattering detector Model 2040, and a Viscotek (Houston, Tex.) 150R 4-capillary solution viscometer. A GPC with the last two independent detectors and at least one of the first detectors is sometimes referred to as "3D-GPC", while the term "GPC" alone generally refers to conventional GPC. Depending on the sample, either the 15-degree angle or the 90-degree angle of the light scattering detector was used for calculation purposes.

Data collection was performed using Viscotek TriSEC software, Version 3, and a 4-channel Viscotek Data Manager DM400. The system was equipped with an on-line solvent degassing device from Polymer Laboratories (Shropshire, UK). Suitable high temperature GPC columns could be used, such as four 30 cm long Shodex HT803 13 micron columns or four 30 cm Polymer Labs columns of 20-μm (micron) mixed-pore-size packing (MixA LS, Polymer Labs). The sample carousel compartment was operated at 140 °C and the column compartment was operated at 150 °C. The samples were prepared at a concentration of 0.1 grams of polymer in 50 milliliters of solvent. The chromatographic solvent and the sample preparation solvent contain 200 ppm of butylated hydroxytoluene (BHT). Both solvents were sparged with nitrogen. The polyethylene samples were gently stirred at 160 °C for four hours (4 h). The injection volume was 200 microliters (μL). The flow rate through the GPC was set at 1 mL/minute.

*NMR ($^{13}C$ and $^1H$):*

NMR analysis was performed at room temperature using a standard NMR solvent, such as chloroform or benzene, and data was acquired on a Varian 500 MHz spectrometer.

*GCMS:*

Tandem gas chromatography/low resolution mass spectroscopy using electron impact ionization (EI) is performed at 70 eV on an Agilent Technologies 6890N series gas chromatograph equipped with an Agilent Technologies 5975 inert XL mass selective detector and an Agilent Technologies Capillary column (HP1MS, 15m X 0.25mm, 0.25 μm (micron)) with respect to the following:
Programed method:

Oven Equilibration Time at 50 °C for 0.5 min
then 25 °C/min to 200 °C, and hold for 5 min

Run Time 11 min

EXAMPLES

Unless stated otherwise, all materials and reagents are commercially available, such as from Sigma Aldrich.

**Example 1**

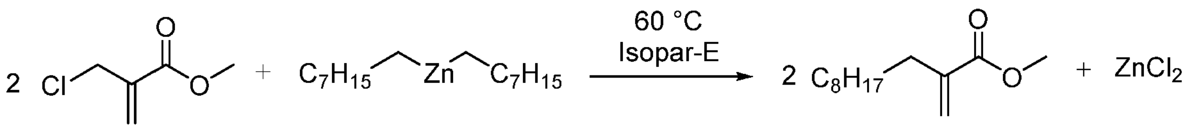


The reaction of Example 1 was performed under an inert nitrogen atmosphere glovebox and in accordance with the above reaction scheme which is exemplary and non-limiting. 5.88 mL of a 0.30 M dioctylzinc solution in Isopar™ E (1.76 mmol) was added to a 20 mL vial. The solution was heated to 60 °C. 0.500 g methyl 2-(chloromethyl)acrylate (3.72 mmol, 2 equiv.) was added dropwise to the hot dioctylzinc solution. Over the course of the slow addition, the solution turned from light brown to clear and became cloudy with a visible white precipitate. After several minutes, the precipitate settled as a sticky yellow residue on the bottom of the vial. After 48 hours at 60 °C, 83 mg of hexamethylbenzene (0.511 mmol) was added as an NMR internal standard. The NMR conversion was calculated to be 62.6%. NMR analysis is shown in FIGS. 1A and 1B. As seen in FIG. 1C and 1D, GC-MS of a reaction aliquot showed formation of the desired product (lower retention time peaks correspond to Isopar™ E). The reaction was quenched with water. Purification to remove Zn salts and the internal standard was carried out by column chromatography eluting with a 2% ethyl acetate in hexanes mixture. 405 mg of product was isolated (51%).

**Example 2**

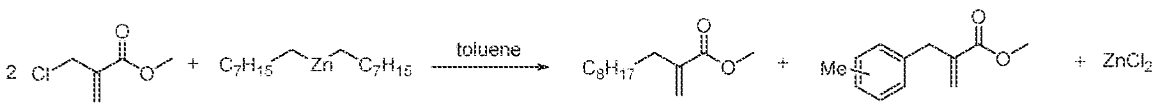


The reaction of Example 2 was performed under an inert nitrogen atmosphere glovebox and in accordance with the above reaction scheme which is exemplary and non-limiting. 7.5 mL of a 0.35 M dioctylzinc solution in toluene (2.63 mmol) was added to a 20 mL vial. The solution was heated to 100 °C. 0.700 g methyl 2-(chloromethyl)acrylate (5.202 mmol, 2 equiv.) was added dropwise to the hot dioctylzinc solution. After 12 hours, the reaction was quenched and the mixture was purified by column chromatography eluting with a 2% ethyl acetate in hexanes mixture. NMR (FIG. 2) and GC-MS analyses confirmed formation of the desired $\alpha$-(alkyl)acrylate.

**Example 3**

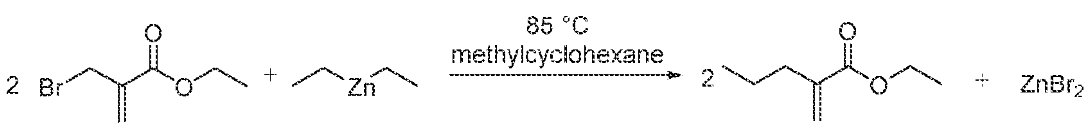


The reaction of Example 3 was performed under an inert nitrogen atmosphere glovebox and in accordance with the above reaction scheme which is exemplary and non-limiting. To the solution of ethyl bromomethyl acrylate (193 mg) in dry methylcyclohexane (5 mL) in a 20 mL vial neat DEZ (61.8 mg, 0.5 equiv) was slowly added at RT. Reaction mixture turned slightly yellow, after overnight at room temperature color disappeared.

After 12 hours at room temperature the NMR conversion was calculated to be 85% as seen in FIG. 3A.

The reaction mixture was additionally heated for 5 hours at 85°C. NMR showed full conversion with the formation of ca. 12 % of unidentified byproduct as seen in FIG. 3B.

**Example 4**

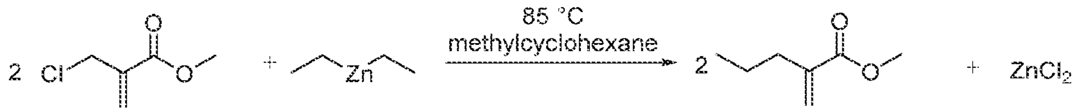


The reaction of Example 4 was performed under an inert nitrogen atmosphere glovebox and in accordance with the above reaction scheme which is exemplary and non-limiting. To the solution of methyl chloromethyl acrylate (134.6 mg) in dry methylcyclohexane (5 mL) in a 20 mL vial neat DEZ (61.8. 0.5 equiv) was slowly added at RT. Reaction mixture turned slightly yellow, after overnight at room temperature color disappeared. 1,3,5-tribromobenzene was added (38.1 mg, 0.121 mmol) was added as internal standard. After 12 hours at room temperature the NMR conversion was calculated to be 77% as seen in FIG. 4A. Reaction mixture was additionally heated for 5 hours at 85°C. NMR showed full conversion as seen in FIG. 4B. Integration *vs*. internal standard gave 95% NMR yield.

## Claims

**1.** A process for preparing an alpha-substituted acrylate, the process comprising:

a) combining starting materials comprising an alpha-(halomethyl) acrylate and an organozinc compound of the formula $R_2Zn$,
thereby forming a product comprising the alpha-substituted acrylate, wherein:

the alpha-substituted acrylate has the formula (I):

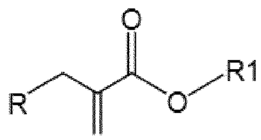
(I);

the alpha-(halomethyl) acrylate has the formula (II):

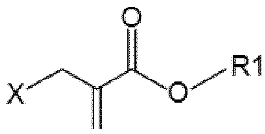
(II);

each R independently is a C1-C26 hydrocarbyl group or a polyolefinyl group;
each R1 independently is a C1-C30 hydrocarbyl group; and
X is a halogen, wherein the process excludes any transmetallation step or reaction.

**2.** The process of claim 1, wherein the starting materials of step a) further comprise a solvent.

**3.** The process of claim 2, wherein the solvent is a non-aromatic hydrocarbon solvent.

**4.** The process of any of the previous claims, wherein the starting materials of step a) further comprise an organocatalyst.

**5.** The process of any of the previous claims, wherein each R independently is a C1-C26 hydrocarbyl group.

**6.** The process of any of claims 1- 4, where each R independently is a polyolefinyl group, which can be defined by the properties of R-H, wherein R-H has a number average molecular weight of greater than 365 g/mol.

**7.** The process of claim 6, wherein the polyolefinyl group is an ethylene-based polymeryl group.

**8.** The process of claim 7, wherein the polyolefinyl group is an ethylene homopolymeryl group comprising units derived from ethylene.

**9.** The process of claim 7, wherein the polyolefinyl group is an ethylene/alpha-olefin interpolymeryl gruop comprising units derived from ethylene and a C3-C30 alpha-olefin.

**10.** The process of claim 6, wherein the polyolefinyl group is a propylene-based polymeryl group.

**11.** The process of claim 10, wherein the polyolefinyl group is a propylene homopolymeryl group comprising units derived from propylene.

**12.** The process of claim 10, wherein the polyolefinyl group is a propylene/alpha-olefin interpolymeryl group comprising units derived from propylene and either ethylene or a C4-C30 alpha-olefin.

**Patentansprüche**

**1.** Verfahren zur Herstellung eines α-substituierten Acrylats, das Verfahren umfassend:

a) Kombinieren von Ausgangsmaterialien, die ein α-(Halogenmethyl)acrylat und eine Organozinkverbindung der Formel $R_2Zn$ umfassen,
wodurch ein Produkt gebildet wird, das das α-substituierte Acrylat umfasst, wobei:

das α-substituierte Acrylat die Formel (I) hat:

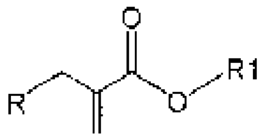
(I);

das α-(Halogenmethyl)acrylat die Formel (II) hat:

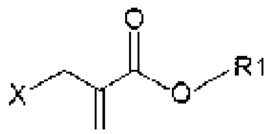
(II);

jedes R unabhängig eine C1-C26-Hydrocarbylgruppe oder eine Polyolefingruppe ist;
jedes R1 unabhängig eine C1-C30-Hydrocarbylgruppe ist; und
X ein Halogen ist, wobei das Verfahren jeglichen Transmetallierungsschritt oder jegliche Transmetallierungsreaktion ausschließt.

**2.** Verfahren nach Anspruch 1, wobei die Ausgangsmaterialien von Schritt a) ferner ein Lösungsmittel umfassen.

**3.** Verfahren nach Anspruch 2, wobei das Lösungsmittel ein nichtaromatisches Kohlenwasserstofflösungsmittel ist.

**4.** Verfahren nach einem der vorstehenden Ansprüche, wobei die Ausgangsmaterialien von Schritt a) ferner einen Organokatalysator umfassen.

**5.** Verfahren nach einem der vorstehenden Ansprüche, wobei jedes R unabhängig eine C1-C26-Hydrocarbylgruppe ist.

**6.** Verfahren nach einem der Ansprüche 1 bis 4, wobei jedes R unabhängig eine Polyolefingruppe ist, die durch die Eigenschaften von R-H definiert werden kann, wobei R-H ein Zahlenmittel des Molekulargewichts von mehr als 365 g/mol aufweist.

**7.** Verfahren nach Anspruch 6, wobei die Polyolefingruppe eine Polymerylgruppe auf Ethylenbasis ist.

**8.** Verfahren nach Anspruch 7, wobei die Polyolefingruppe eine Ethylenhomopolymerylgruppe ist, die von Ethylen abgeleitete Einheiten umfasst.

**9.** Verfahren nach Anspruch 7, wobei die Polyolefingruppe eine Ethylen/$\alpha$-Olefin-Interpolymerylgruppe ist, die Einheiten umfasst, die von Ethylen und einem C3-C30-$\alpha$-Olefin abgeleitet sind.

**10.** Verfahren nach Anspruch 6, wobei die Polyolefingruppe eine Polymerylgruppe auf Propylenbasis ist.

**11.** Verfahren nach Anspruch 10, wobei die Polyolefingruppe eine Propylenhomopolymerylgruppe ist, die von Propylen abgeleitete Einheiten umfasst.

**12.** Verfahren nach Anspruch 10, wobei die Polyolefingruppe eine Propylen/$\alpha$-Olefin-Interpolymergruppe ist, die Einheiten umfasst, die von Propylen und entweder Ethylen oder einem C4-C30-$\alpha$-Olefin abgeleitet sind.

**Revendications**

**1.** Procédé pour la préparation d'un acrylate à substitution alpha, le procédé comprenant :

a) la combinaison de matières de départ comprenant un acrylate d'alpha-(halométhyle) et un composé organozinc de formule $R_2Zn$,
permettant ainsi de former un produit comprenant l'acrylate à substitution alpha, dans lequel :

l'acrylate à substitution alpha a la formule (I) :

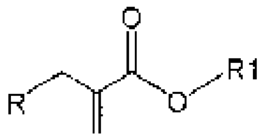
(I);

l'acrylate d'alpha-(halométhyle) a la formule (II) :

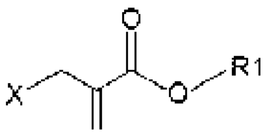
(II);

chaque R est indépendamment un groupe hydrocarbyle en C1-C26 ou un groupe polyoléfinyle ;
chaque R1 est indépendamment un groupe hydrocarbyle en C1-C30 ; et
X est un halogène, dans lequel le procédé exclut toute étape ou réaction de transmétallation.

**2.** Procédé selon la revendication 1, dans lequel les matières de départ de l'étape a) comprennent en outre un solvant.

**3.** Procédé selon la revendication 2, dans lequel le solvant est un solvant hydrocarboné non aromatique.

**4.** Procédé selon l'une quelconque des revendications précédentes, dans lequel les matières de départ de l'étape a) comprennent en outre un organocatalyseur.

**5.** Procédé selon l'une quelconque des revendications précédentes, dans lequel chaque R est indépendamment un groupe hydrocarbyle en C1-C26.

**6.** Procédé selon l'une quelconque des revendications 1 à 4, dans lequel chaque R est indépendamment un groupe polyoléfinyle qui peut être défini par les propriétés de R-H, dans lequel R-H a une masse moléculaire moyenne en nombre supérieure à 365 g/mol.

**7.** Procédé selon la revendication 6, dans lequel le groupe polyoléfinyle est un groupe polyméryle à base d'éthylène.

8. Procédé selon la revendication 7, dans lequel le groupe polyoléfinyle est un groupe homopolyméryle d'éthylène comprenant des motifs dérivés de l'éthylène.

9. Procédé selon la revendication 7, dans lequel le groupe polyoléfinyle est un groupe interpolyméryle éthylène/alpha-oléfine comprenant des motifs dérivés de l'éthylène et d'une alpha-oléfine en C3-C30.

10. Procédé selon la revendication 6, dans lequel le groupe polyoléfinyle est un groupe polyméryle à base de propylène.

11. Procédé selon la revendication 10, dans lequel le groupe polyoléfinyle est un groupe homopolyméryle de propylène comprenant des motifs dérivés du propylène.

12. Procédé selon la revendication 10, dans lequel le groupe polyoléfinyle est un groupe interpolyméryle propylène/alpha-oléfine comprenant des motifs dérivés du propylène et soit de l'éthylène, soit d'une alpha-oléfine en C4-C30.

FIG. 1A

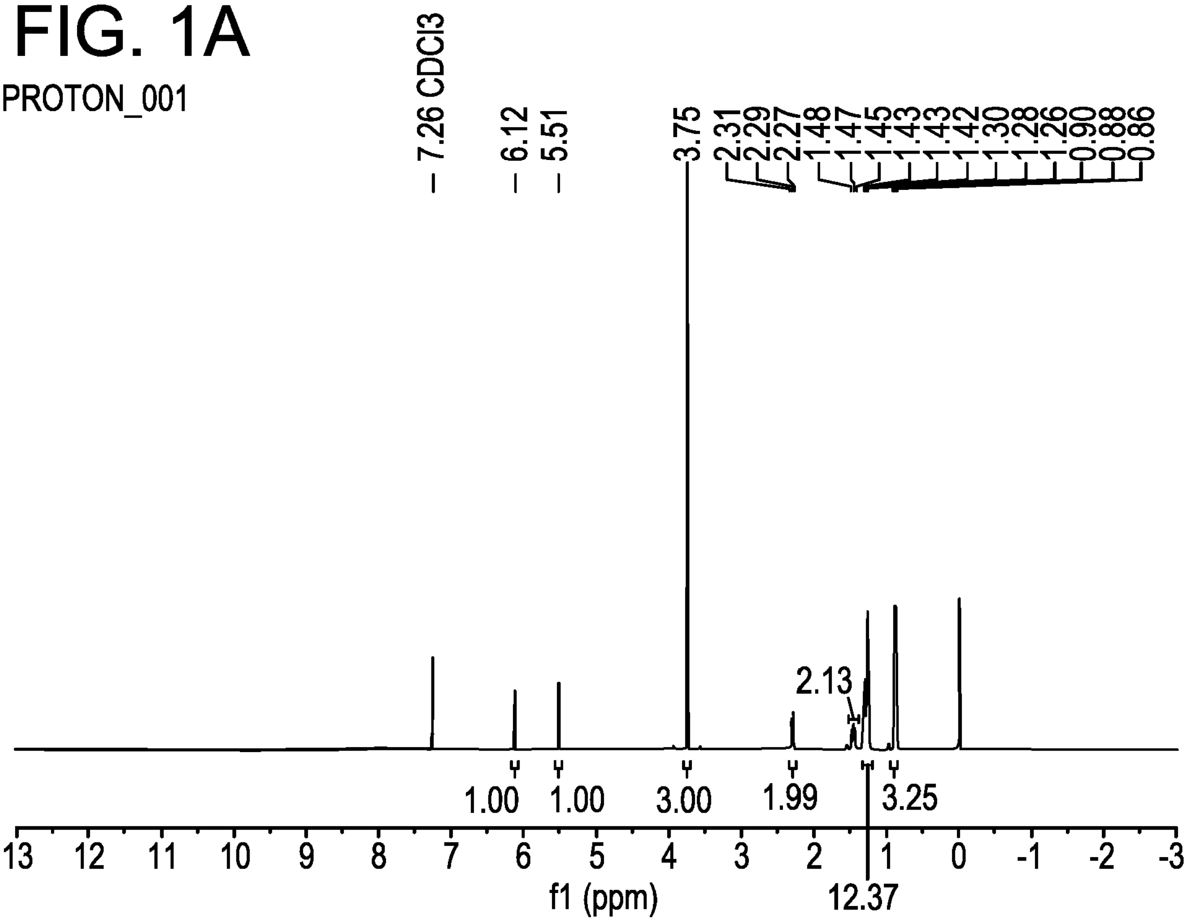
PROTON_001
7.26 CDCl3
6.12
5.51
3.75
2.31
2.29
2.27
1.48
1.47
1.45
1.43
1.43
1.42
1.30
1.28
1.26
0.90
0.88
0.86
2.13
1.00
1.00
3.00
1.99
3.25
12.37
13 12 11 10 9 8 7 6 5 4 3 2 1 0 -1 -2 -3
f1 (ppm)

FIG. 1B

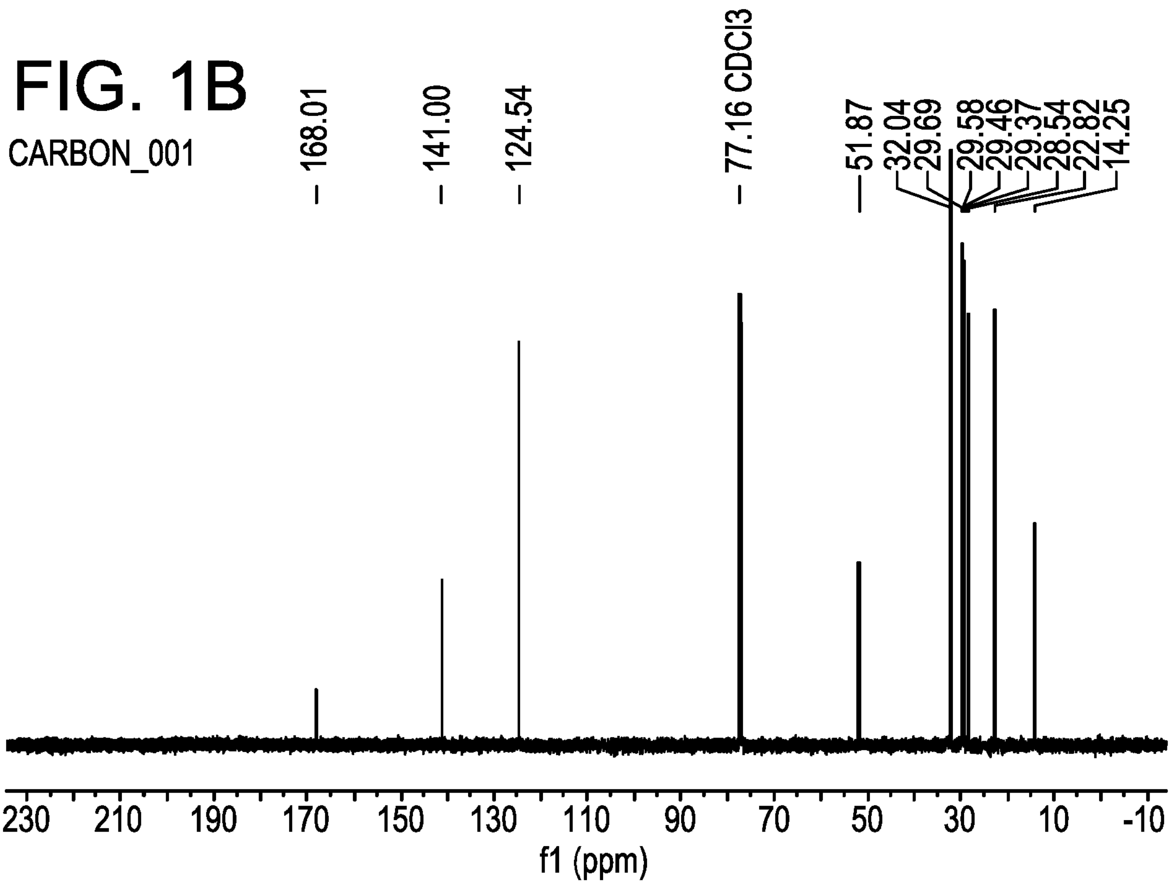
CARBON_001
168.01
141.00
124.54
77.16 CDCl3
51.87
32.04
29.69
29.58
29.46
29.37
28.54
22.82
14.25
230 210 190 170 150 130 110 90 70 50 30 10 -10
f1 (ppm)

# FIG. 1C

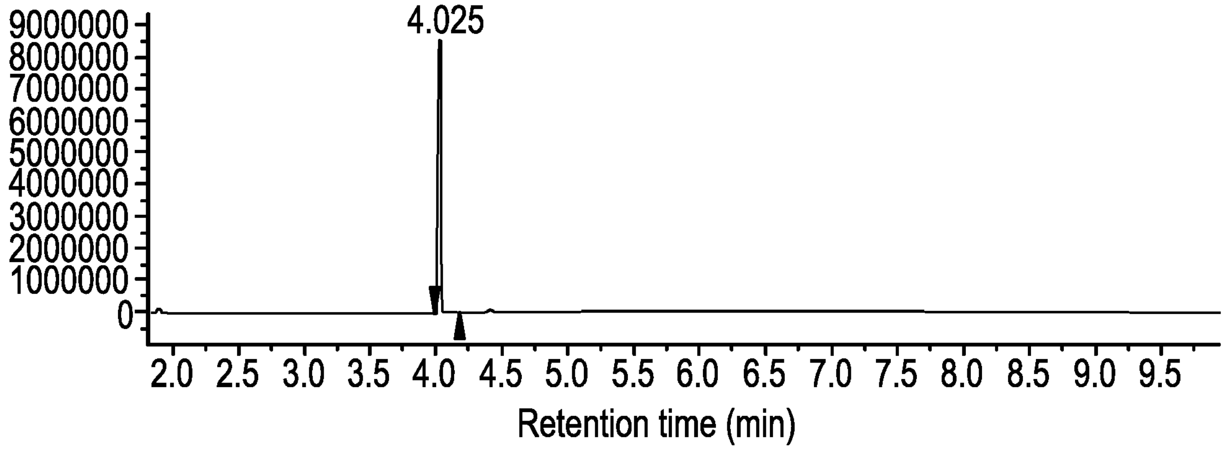
4.025
9000000
8000000
7000000
6000000
5000000
4000000
3000000
2000000
1000000
0
2.0 2.5 3.0 3.5 4.0 4.5 5.0 5.5 6.0 6.5 7.0 7.5 8.0 8.5 9.0 9.5
Retention time (min)

# FIG. 1D

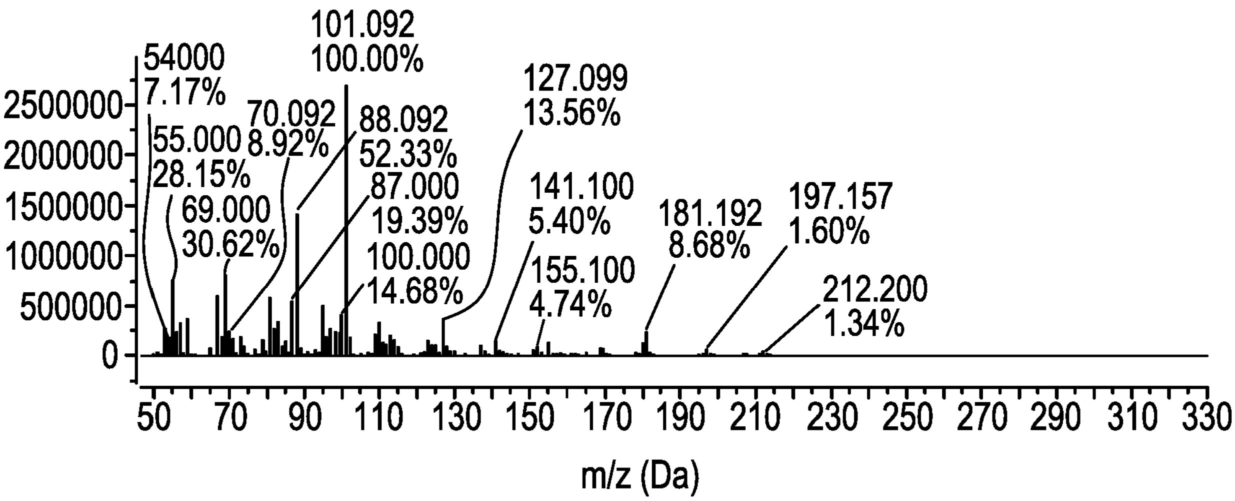
101.092
100.00%
54000
7.17%
55.000
28.15%
69.000
30.62%
70.092
8.92%
88.092
52.33%
87.000
19.39%
100.000
14.68%
127.099
13.56%
141.100
5.40%
155.100
4.74%
181.192
8.68%
197.157
1.60%
212.200
1.34%
2500000
2000000
1500000
1000000
500000
0
50 70 90 110 130 150 170 190 210 230 250 270 290 310 330
m/z (Da)

FIG. 2

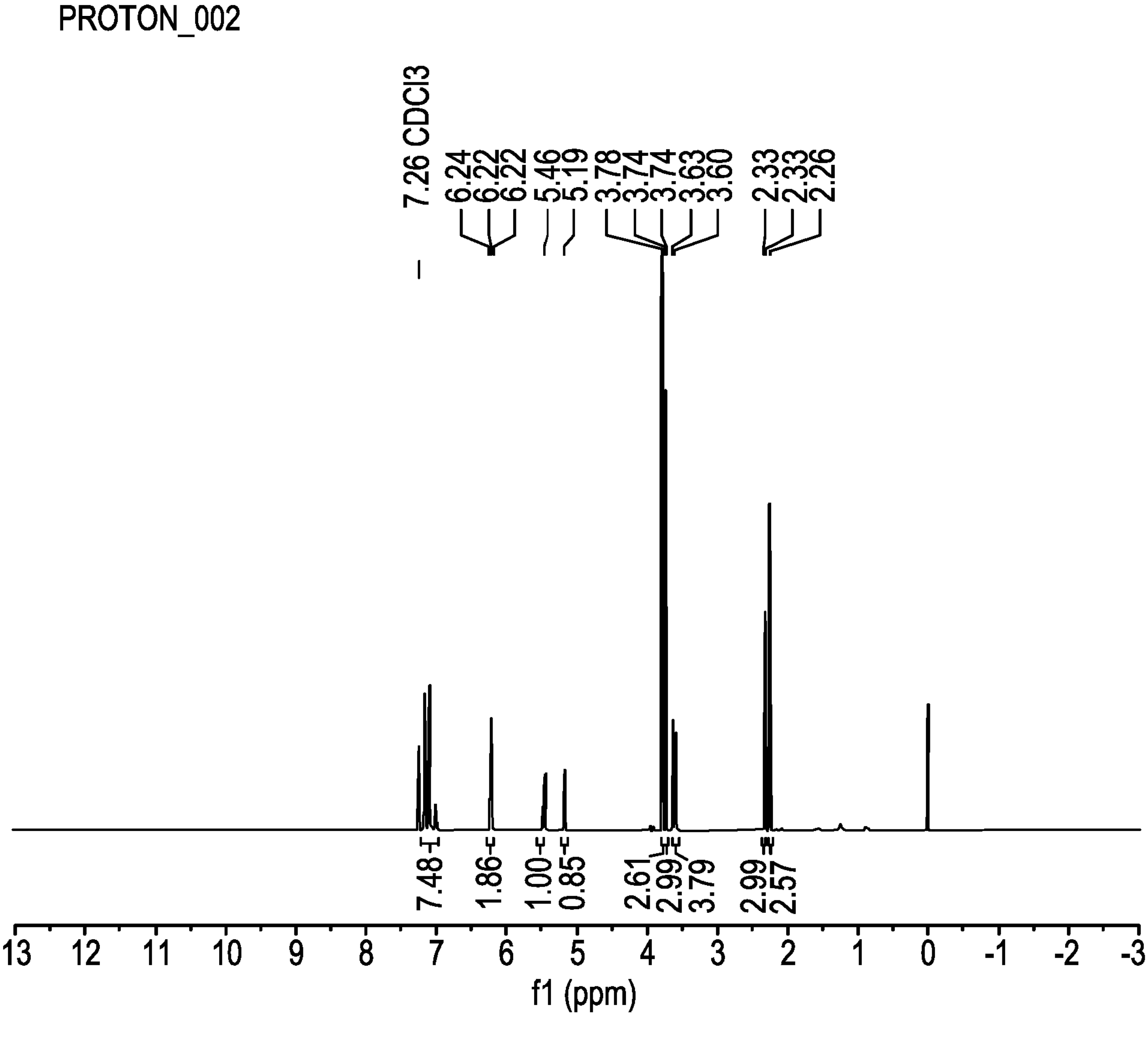
PROTON_002
7.26 CDCl3
6.24
6.22
6.22
5.46
5.19
3.78
3.74
3.74
3.63
3.60
2.33
2.33
2.26
7.48
1.86
1.00
0.85
2.61
2.99
3.79
2.99
2.57
13 12 11 10 9 8 7 6 5 4 3 2 1 0 -1 -2 -3
f1 (ppm)

FIG. 3A

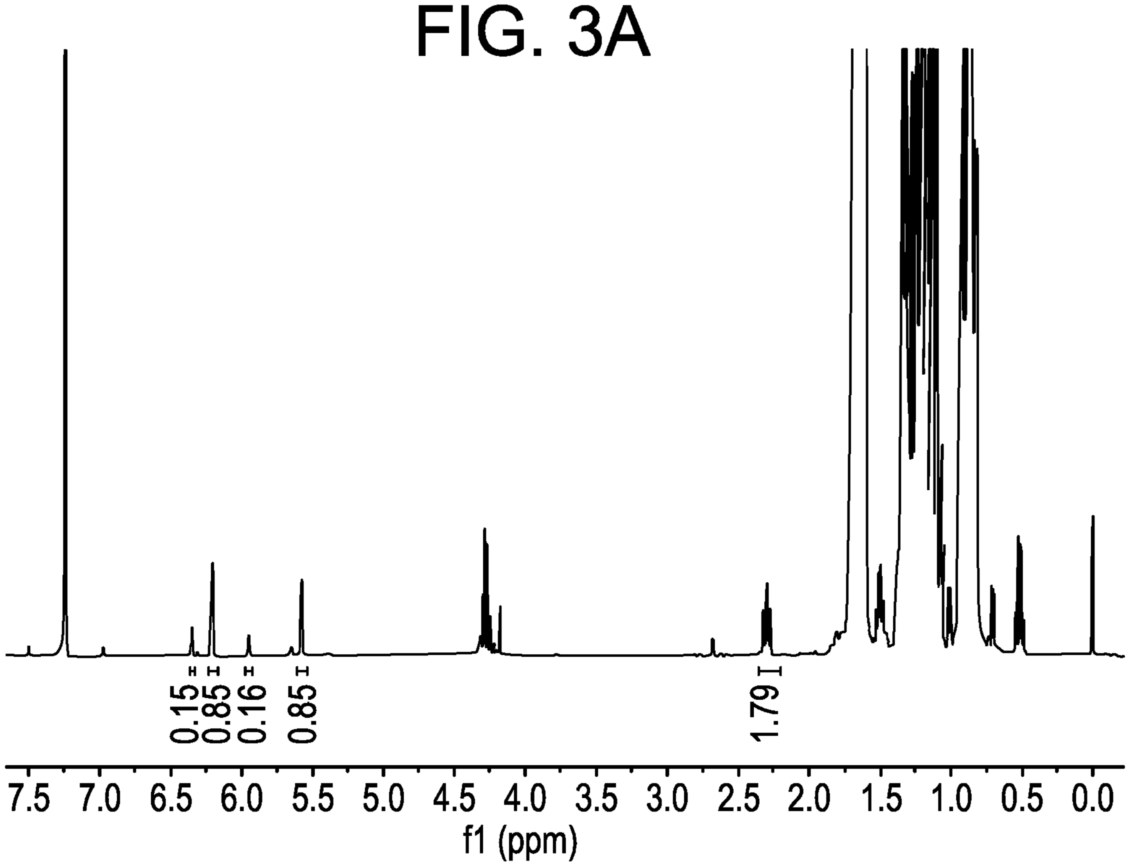
0.15
0.85
0.16
0.85
1.79
7.5 7.0 6.5 6.0 5.5 5.0 4.5 4.0 3.5 3.0 2.5 2.0 1.5 1.0 0.5 0.0
f1 (ppm)

FIG. 3B

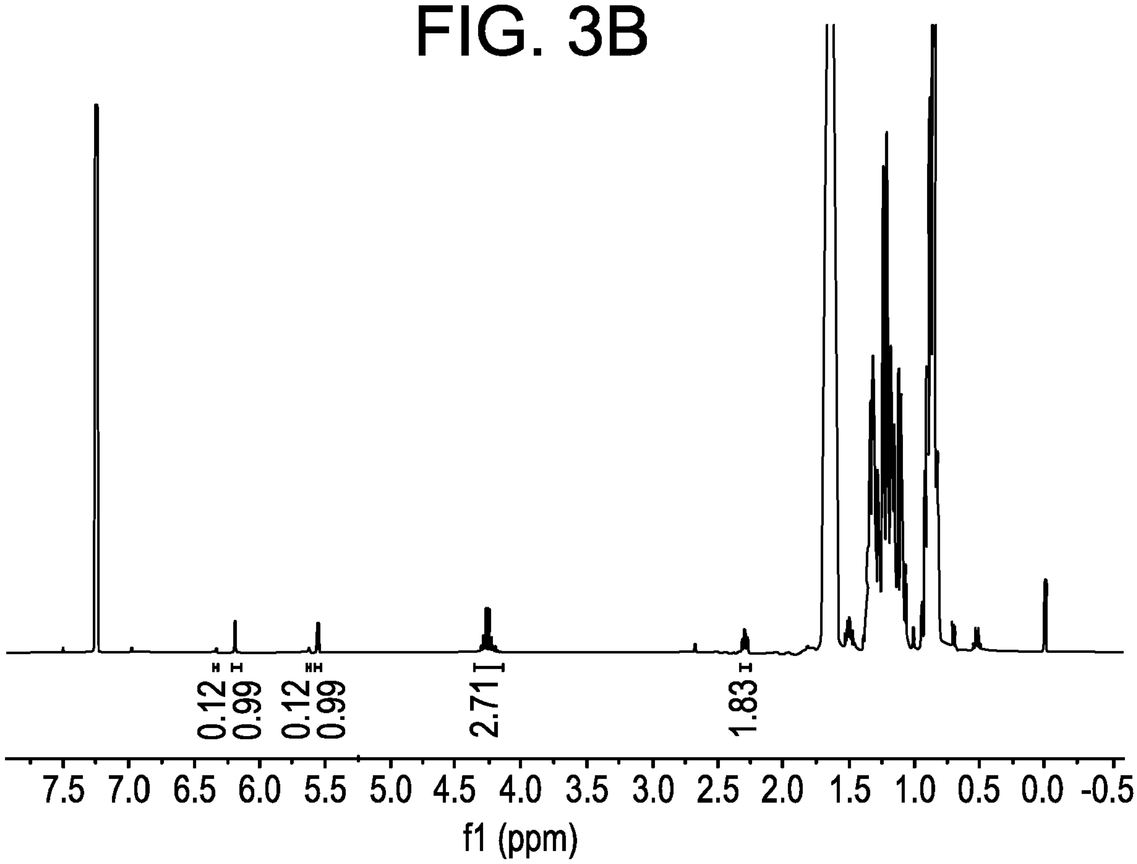
0.12
0.99
0.12
0.99
2.71
1.83
7.5 7.0 6.5 6.0 5.5 5.0 4.5 4.0 3.5 3.0 2.5 2.0 1.5 1.0 0.5 0.0 -0.5
f1 (ppm)

FIG. 4A

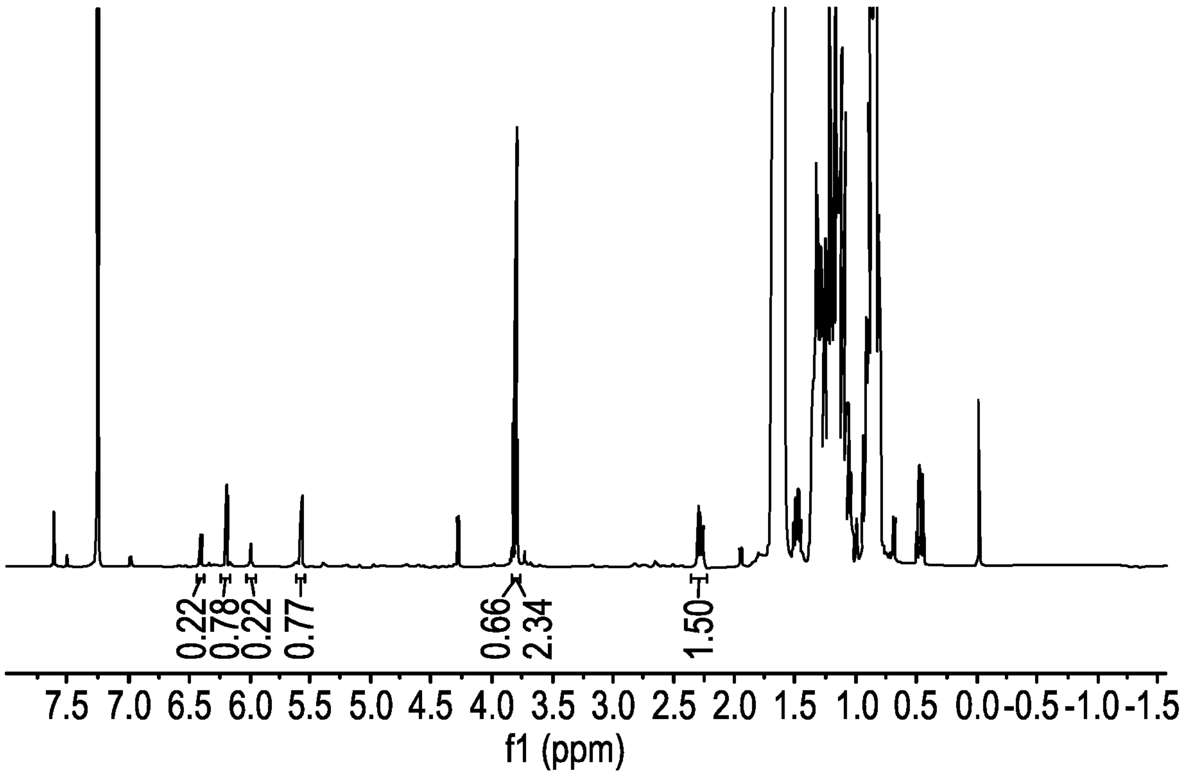
0.22
0.78
0.22
0.77
0.66
2.34
1.50
7.5 7.0 6.5 6.0 5.5 5.0 4.5 4.0 3.5 3.0 2.5 2.0 1.5 1.0 0.5 0.0 -0.5 -1.0 -1.5
f1 (ppm)

FIG. 4B

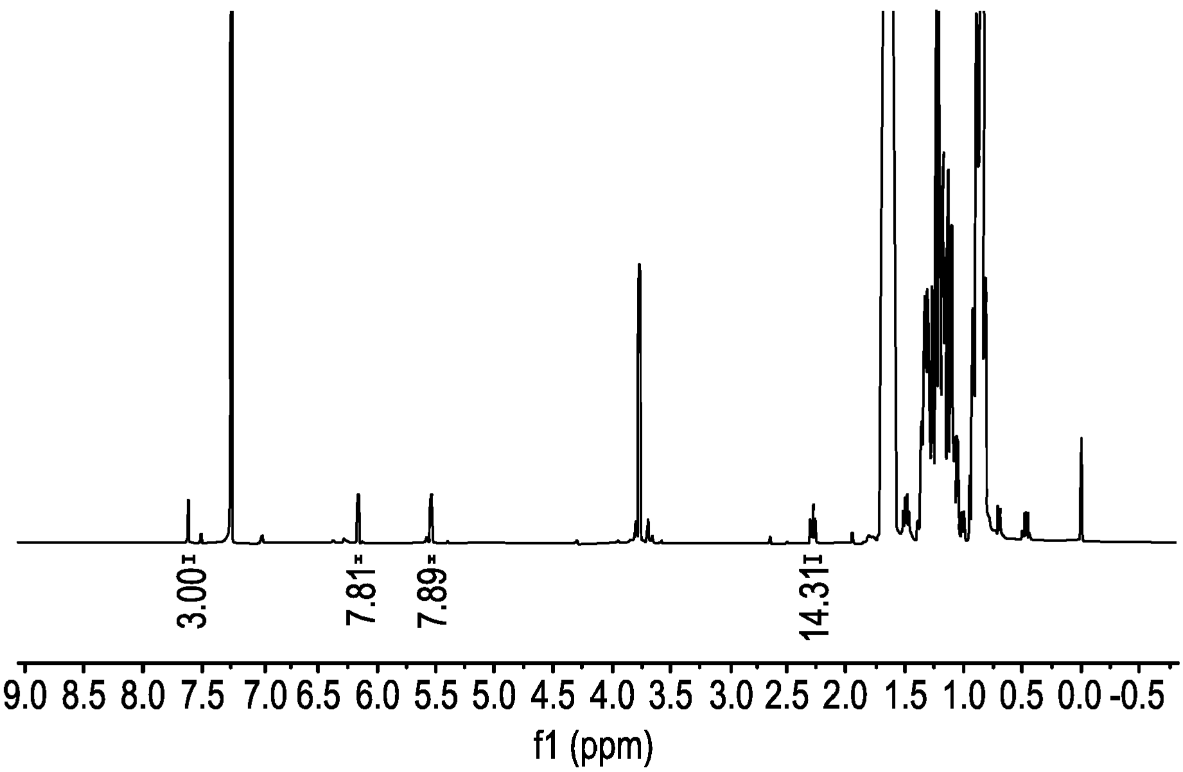
3.00
7.81
7.89
14.31
9.0 8.5 8.0 7.5 7.0 6.5 6.0 5.5 5.0 4.5 4.0 3.5 3.0 2.5 2.0 1.5 1.0 0.5 0.0 -0.5
f1 (ppm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7858706 B2
- US 8198374 B2
- US 8318864 B2
- US 8609779 B2
- US 8710143 B2
- US 8785551 B2
- US 9243090 B2
- US 8686087 B
- US 8716400 B
- WO 2017044547 A
- US 8822598 B2
- WO 201601028961 A1
- US 7858706 B
- US 8053529 B
- WO 2005090426 A
- WO 2005090427 A
- WO 2007035485 A
- WO 2009012215 A
- WO 2014105411 A
- WO 2017173080 A
- US 20060199930 A
- US 20070167578 A
- US 20080311812 A
- US 7355089 B2
- US 8058373 B2
- US 8785554 B2
- US 5783512 A
- WO 2019182992 A


### Non-patent literature cited in the description


- *Tetrahedron Letters*, 13 February 1995, vol. 36 (7), 1023-1026
- Periodic Table of the Elements. CRC Press, Inc, 2003
- **WILLIAMS** ; **WARD**. *J. Polym. Sci., Polym. Let*, 1968, vol. 6, 621